(19) Europäisches Patentamt
European Patent Office
Office européen des brevets

(11) Publication number: 0 391 631 A1

(12) # EUROPEAN PATENT APPLICATION

(21) Application number: 90303473.4

(22) Date of filing: 30.03.90

(51) Int. Cl.5: C07D 215/12, C07D 217/14, C07D 213/57, C07D 239/26, C09B 23/14, G11B 7/24

(30) Priority: 31.03.89 GB 8907311

(43) Date of publication of application: 10.10.90 Bulletin 90/41

(84) Designated Contracting States: CH DE FR GB IT LI

(71) Applicant: NATIONAL RESEARCH DEVELOPMENT CORPORATION
101 Newington Causeway
London SE1 6BU(GB)

(72) Inventor: Ashwell, Geoffrey Joseph
3 Lister Drive
Northampton NN4 9XE(GB)

(74) Representative: Bentham, Stephen et al
J.A. Kemp & Co. 14 South Square Gray's Inn
London WC1R 5EU(GB)

(54) **Photochromic compounds useful in Langmuir-Blodgett films.**

(57) Compounds of formula (I);

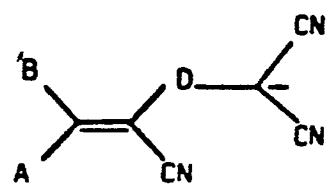

in which A represents certain pyridinium, quinolinium, isoquinolinium or pyrimidinium groups unsubstituted or substituted by F, Cl, Br, CN or $NO_2$ and substituted on nitrogen by a straight hydrocarbon group of at least 6 carbon atoms which is unsubstituted or substituted by fluorine and the chain of which may comprise a group -O-, -S-, -CO-O-, -O-CO-, -NH-CO-, -CO-NH- or carbon to carbon double or triple bond, or R represents a phenyl group substituted in the 3- or 4-position by a group R as hereinbefore defined or by a corresponding group containing less than 6 carbon atoms;
B is H, $CH_3$, F, Cl, Br, CN or $NO_2$; and D represents certain phenyl or naphthyl groups are photochromic compounds.

EP 0 391 631 A1

# PHOTOCHROMIC COMPOUNDS USEFUL IN LANGMUIR-BLODGETT FILMS

The present invention relates to compounds useful in Langmuir-Blodgett films, a process for their preparation, Langmuir-Blodgett films containing them and optical memories comprising said Langmuir-Blodgett films.

Photochromism results from a reversible change in the molecular structure or charge distribution of a compound during irradiation and often involves one of the following processes: (i) cis-trans isomerism; (ii) proton transfer; (iii) bond cleavage; or (iv) intermolecular charge transfer. Such compounds can be used in security printing, photochromic lenses and optical data storage.

A high density multifrequency memory consisting of a Langmuir-Blodgett (LB) film of two J-aggregated spiropyrans, such as 1′-octadecyl-3′,3′-dimethyl-6-nitro-8-(docosanoyloxy)methylspiro[2H-1-benzopyran-2,2′-indoline] and 1′-octadecyl-3′,3′-dimethyl-6-nitro-8-methoxyspiro[2H-1-benzopyran-2,2′-indoline] has been proposed by Ando et al in Thin Solid Films 160 279 (1988) and EP-A-193931. Three-dimensional data storage is possible if layers can be switched at different wavelengths, but to store several bits per pixel requires molecules with switchable non-overlapping bands. The LB absorption spectra of many of the known photochromic materials, for example viologens, thioindigos, salicylidene anils and azobenzenes, are broad and, so far, only the spiropyrans have shown any promise for use in a multifrequency device. Even so, for the mixed monolayer assemblies of Ando's spiropyrans the J-band maxima occur at 600 and 618 nm with half widths at half maximum of 50 and 25 nm respectively. Thus, the resolution is poor. There is a need for alternative photochromic materials with sharper non-overlapping bands.

Metzger, Heimer and Ashwell in Mol. Cryst. Liq. Cryst. 107, 133 (1984) and Akhtar, Tanaka, Metzger and Ashwell in Mol. Cryst. Liq. Cryst. 139, 353 (1986) disclose Z-β-(N-methyl-2-pyridyl)-α-cyano-4-styryl dicyanomethanide (P3CNQ).

The present invention provides a compound of formula (I).

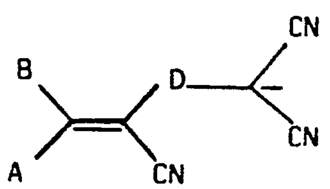

in which

A is

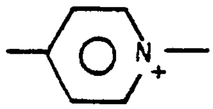 IIA 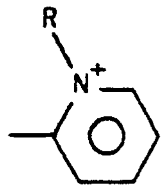 IIB 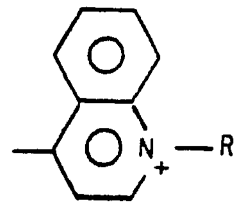 IIC

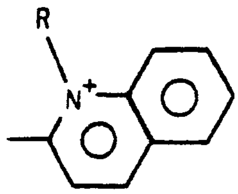 IID 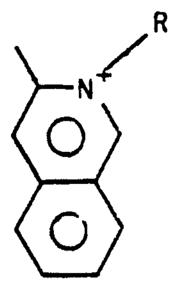 IIE 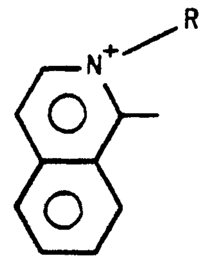 IIF or

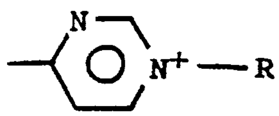 IIG

in which the pyridinium, quinolinium, isoquinolinium or pyrimidinium group is unsubstituted or substituted by at least one of F, Cl, Br, CN or $NO_2$ and R represents a straight hydrocarbon group containing at least 6 carbon atoms, preferably containing from 6 to 30 (more preferably 6 to 22 or 6 to 20) carbon atoms which is unsubstituted or substituted by fluorine and the chain of which may comprise a group -O-, -S-, -CO-O-, -O-CO-, -HN-CO-, -CO-NH- or carbon to carbon double or triple bond, or R represents a group $R^1$ which is a phenyl group substituted in the 3- or, more preferably, 4-position by a group R as hereinbefore defined or by a corresponding group containing less than 6 (preferably 4 or more) carbon atoms;
B is H, $CH_3$, F, Cl, Br, CN or $NO_2$: and
D is

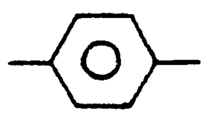 , 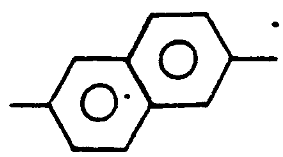 or 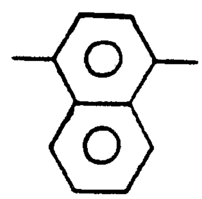

in which the phenyl group is unsubstituted or substituted by at least one of F, Cl, Br, CN or alkoxy of 1 to 4 carbon atoms, preferably methoxy. The compounds of formula I are photochromic and useful in Langmuir Blodgett films which can be photochromically switched.

Preferred compounds are those in which the pyridinium, quinolinium, isoquinolinium or pyrimidinium group is unsubstituted or substituted by at least one F atom, B is H or CN and/or D is a phenyl group unsubstituted or substituted by at least one, for example four, F atoms or is a naphthyl group.

R is a hydrophobic group and may, for example, be an alkyl group or a hydrocarbon group which may contain, e.g. one or more double or triple carbon-carbon bonds. It is straight to avoid interference with packing in LB films.

Compounds in which A is a pyridinium group are referred to hereinafter as P3CNQ compounds and those in which A is a quinolinium or isoquinolinium group as Q3CNQ compounds.

Figure 1 shows the possible intramolecular change in a compound of formula (I) on switching as well as possible bond rotations. Figures 2 and 3 show the uv/visible LB film spectra of $C_{16}H_{33}$-P3CNQ before and after switching (the pyridinium ring is of formula IIA).

In the compounds of formula (I), the photochromic process probably involves electron transfer from the negatively charged dicyanomethanide group to the positively charged heterocycle. The compounds of

formula (I) have sharp LB bands, and can therefore be used in three-dimensional data storage devices.

The compounds of formula (I) are believed to exist as the zwitterions depicted which are photochromic and are readily bleached when irradiated at wavelengths which overlap the charge transfer bands. Referring by way of example to the compounds of formula (I) in which A represents a group of formula IIA or IIB the switching is attributed to intramolecular charge transfer from the negatively charged dicyanomethanide group to the positively charged pyridinium ring. This necessitates not only a change in the bond alternation but also rotation of the C-B and C-C≡N groups of the -CB = C(CN)- bridge into the planes of the pyridinium donor and phenyldicyanomethanide acceptor respectively. A common feature of photochromically switched materials is thermal reversion to the preswitched form and, in the dark, the bleached solutions of compounds of formula (I) slowly recolour. In contrast, the bleached LB films are stable and their spectra do not alter even after one year.

The intramolecular charge transfer band of $C_{16}H_{33}$-P3CNQ (in which the pyridinium group is of formula IIA) in acetonitrile occurs at 645 nm with $\epsilon$ = 3900 $mol^{-1}m^2$ whereas for the LB film it is blue shifted to 495 nm with an absorbance of 0.020 monolayer $^{-1}$ (Figure 2). The 150 nm blue shift is not unusual and a similar change from 710 nm (acetonitrile) to 565 nm (LB film) has been obtained for $C_{16}H_{33}$-Q3CNQ (in which the quinolinium group is of formula IIC) (Figure 4). A novel feature, however, is the intense narrowing of the LB intramolecular charge transfer bands. For $C_{16}H_{33}$-P3CNQ the half width at half maximum is 27 nm compared with the solution value of 76 nm whereas for $C_{16}H_{33}$-Q3CNQ the corresponding half widths are 22 and 104 nm respectively, as shown in Table 1.

TABLE 1

| Spectroscopic data | | | | | | |
|---|---|---|---|---|---|---|
| | Acetonitrile | | | LB film | | |
| Zwitterion | $\lambda_{max}$/nm | HWHM/nm | $\epsilon/m^2mol^{-1}$ | $\lambda_{max}$/nm | HWHM/nm | A* |
| $C_{16}H_{33}$-P3CNQ | 645 | 76 | 3900 | 495 | 27 | 0.020 |
| $C_{16}H_{33}$-Q3CNQ | 712 | 104 | 4000 | 565 | 22 | 0.020 |

*Absorbance per monolayer.
HWHM = half width at half maximum.

The LB bands are extremely sharp and in the region of their absorption maxima there is little direct overlap.

For compounds of formula (I) in which A is a pyridinium group of formula IIA and R is a straight hydrocarbon group containing 6 carbon atoms and in which A is a quinolinium group of formula IIC and R is a straight hydrocarbon group containing from 6 to 14 carbon atoms the switchable bands are red shifted to 634 nm and 614 nm, respectively. The LB isotherms give smaller areas per molecule at the deposition pressure and thus it is believed that this band arises from intermolecular charge transfer. The intramolecular and intermolecular charge transfer bands of the R-Q3CNQ (in which the quinolinium group is of formula IIC) homologues are shown in Figures 4 and 5 respectively.

The pyridinium compounds of general formula (I) in which R contains 6 carbon atoms and the quinolinium compounds in which R contains 6 to 14 carbon atoms generally show only an intermolecular LB band: pyridinium compounds in which R contains at least 7 carbon atoms and quinolinium compounds in which R contains at least 15 carbon atoms generally show only intramolecular LB bands.

The compounds of formula (I) are suitable for multifrequency switching and the charge transfer band wavelengths may be finely tuned to optimise their use as components of a three-dimensional optical memory. The wavelength is dependent, in part, upon the difference between the ionisation energy of the donor end and the electron affinity of the acceptor end. Thus, the 70 nm shift from 495 nm for $C_{16}H_{33}$-P3CNQ (in which the pyridinium group is of formula IIA) to 565 nm for $C_{15}H_{33}$- Q3CNQ (in which the quinolinium group is of formula IIC) is consistent with the quinolinium cation being a stronger electron acceptor. Electron withdrawing substituents at the heterocyclic end cause the charge transfer band to be red shifted whereas those at the phenyldicyanomethanide end cause it to be blue shifted. Thus, as the position of the absorption band is dependent on the difference between the ionisation energy of the donor end and the electron affinity of the acceptor end it may be finely tuned by the use of various substituents.

According to a further feature of the present invention the compounds of general formula (I) are

prepared by a process which comprises the reaction of a salt, preferably a halide, of the general formula:

**A - $CH_2$ - B . X**

(wherein X represents an anion, preferably a halide, preferably the bromide or iodide, and A and B are as hereinbefore defined) and a compound of the general formula:

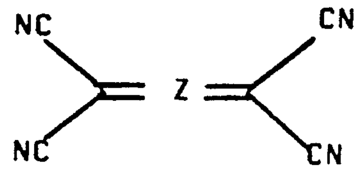

III

wherein Z represents:

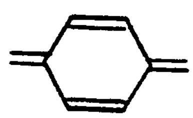

,

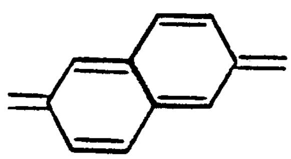

or

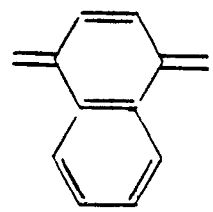

or an alkali metal salt (preferably the lithium salt) thereof.

The reaction is generally carried out in an organic solvent, e.g. acetonitrile when an alkali metal salt is used, or methanol, which is preferably anhydrous. The reaction is preferably conducted at the reflux temperature of the reaction mixture. When a compound of general formula III is used (and not an alkali metal salt thereof) a base, e.g. piperidine or N-methylpiperidine, should be present.

The compounds of general formula

A - $CH_2$ - B . X wherein R in the group A represents a group $R^1$ as hereinbefore defined may be prepared by adaptation of the method described in British Patent Specification no. 1514466. An N-(2,4-dinitrophenyl) salt of the corresponding heterocyclic compound is reacted with an aniline substituted in the 3- or, preferably 4-, position by a group R as hereinbefore defined or by a corresponding group containing less than 6 (preferably 4 or more) carbon atoms to introduce onto the nitrogen atom of the group A a group $R^1$ as hereinbefore defined.

The starting materials for use in the process of the invention are known or can be prepared by the application or adaptation of known methods.

The present invention also provides a Langmuir-Blodgett film which comprises a compound of formula (I) and a multilayer film comprising two or more Langmuir-Blodgett films, each of which comprises a compound of formula (I) having a different switching frequency from the other compound(s) of formula (I).

LB films of the compounds of formula (I) may be produced by known methods, for example, depositing the compounds of formula (I) on a suitable substrate, for example hydrophilically treated quartz slides using a LB film forming apparatus, for example a Nima Technology Langmuir-Blodgett trough. The zwitterions may, for example, be dissolved in Aristar grade acetone or dichloromethane and their solutions spread on the pure water subphase of the trough. They can be deposited by raising or lowering the substrate through the floating monolayer. When a hydrophilic substrate such as quartz is used the substrate is preferably raised from the aqueous subphase through the monolayer. During deposition the surface pressure can, for example, be maintained at 25mN $m^{-1}$ and the substrate movement at 50 $\mu m\ s^{-1}$. Multilayer films of the

same, or different compounds of formula (I) may be prepared by repeating the procedure.

Langmuir-Blodgett monolayers comprising more than one compound of formula (I) may also be used. Certain such films possess particularly advantageous properties. For example, heteromolecular LB films of $C_{16}H_{33}$ P3CNQ (in which the pyridinium group is IIA) and $C_{16}H_{33}$ Q3CNQ (in which the quinolinium group is IIC) exhibit a single, sharp, photochromic, charge transfer band which may be finely tuned within the range 495 to 565 nm with a half width at half maximum of 22 to 34 nm. The variation of the wavelength with the mole proportion of Q3CNQ and the corresponding changes in the half width at half maximum are shown in Figures 6 and 7 respectively. Such tuning can also be achieved with other similar mixtures provided that phase separation does not take place within the monolayer: if such separation does take place separate peaks are observed.

The fact that the absorption is very sharp shows that the resultant band is not a simple combination of two separate charge transfer bands. It also suggests that the zwitterions do not phase separate in the mixed films.

The fact that the photobleachable band is tunable within the range 495 to 565 nm makes it accessible to common laser wavelengths, e.g. to the second harmonic of the Nd:YAG at 532 nm.

The following Examples illustrate the preparation of compounds of formula (I).

## EXAMPLE 1

### Z-β-(1-hexadecyl-4-pyridinium)-α-cyano-4-styryldicyanomethanide

A solution of 1-hexadecyl-4-methylpyridinium bromide (0.40 g, 1 mmol) and $Li^{+}TCNQ^{-}$ (0.21 g, 1 mmol) in dry acetonitrile was heated at reflux until the colour changed from green to blue. After cooling, microcrystalline $C_{16}H_{33}$-P3CNQ was obtained by filtration (0.15 g, 30%). Recrystallisation from acetonitrile gave fine green crystals. $^{1}H$ NMR (DMSO): 0.85 (3H, t, J = 5.5 Hz, $CH_3$-); 1.26 (28H, s, -$(CH_2)_{14}$-); 4.98 (2H, broad s, -$CH_2N^{+}$); 6.85 (2H, d, J = 8.8 Hz, acceptor-H); 7.58 (2H, d, J = 8.8 Hz, acceptor-H); 7.84 (1H, s, -CH=C(CN)-); 8.28 (2H, m, J = 7.1 Hz, donor-H); 8.87 (2H, d, J = 6.8 Hz, donor-H). MS: m/z 494 ($M^{+}$, 30%), 270 ($M^{+}$ - $C_{16}H_{33}$ + H, 90%), 205 ($M^{+}$ - $C_{16}H_{33}$ - $C(CN)_2$, 100%). IR (KBr): 2135, 2175 $cm^{-1}$ (C≡N). UV/vis ($CH_3CN$): $\lambda_{max}$330, 645 nm. Calc. for $C_{33}H_{42}N_4$: C, 80.1; H, 8.6; N, 11.3 %. Found: C, 79.8; H, 8.4; N, 11.1%.

## EXAMPLE 2

### Z-β-(1-hexadecyl-4-quinolinium)-α-cyano-4-styryldicyanomethanide

A solution of 1-hexadecyl-4-methylquinolinium bromide (0.45 g, 1 mmol) and neutral TCNQ (0.20 g, 1 mmol) in dry methanol was treated with piperidine (0.1 ml) and heated at reflux for 24 h. After cooling, microcrystalline $C_{16}H_{33}$-Q3CNQ was obtained by filtration (0.19 g, 35 %). Recrystallisation from acetonitrile gave blue-green crystals. $^{1}H$ NMR (DMSO): 0.87 (3H, t, J = 5.6 Hz, $CH_3$-); 1.29 (28H, s, -$(CH_2)_{14}$-); 5.00 (2H, broad s, -$N^{+}CH_2$-); 6.85 (2H, d, J = 8.1 Hz, acceptor-H); 7.75 (2H, d, J = 8.5 Hz, acceptor-H); 8.10-8.80 (6H, m, donor-H); 9.45 (1H, d, J = 6.3 Hz, donor-H). MS: m/z 544 ($M^{+}$, 75%); 320 ($M^{+}$ - $C_{16}H_{33}$ + H, 90%); 255 ($M^{+}$ - $C_{16}H_{33}$ $C(CN)_2$, 100%). IR (KBr): 2135, 2175 $cm^{-1}$ (C≡N). UV/vis ($CH_3CN$): $\lambda_{max}$348, 378, 712 nm. Calc. for $C_{37}H_{44}N_4$: C, 81.6; H, 8.1; N, 10.3 %. Found: C, 81.3; H, 7.8; N, 10.0 %.

## EXAMPLE 3

The compound:

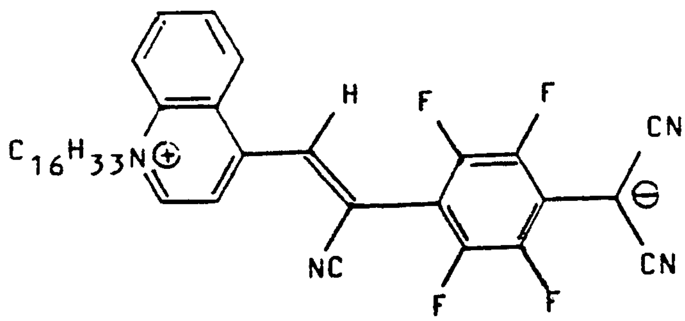

[λmax in acetonitrile = 565 nm compared with 712 nm for the corresponding compound without fluorine substituents;
elemental analysis: calculated C 72.1, H 6.5, N 9.1%; found C 71.7, H 6.1 N 8.8%]
was prepared in a similar manner to that described in Example 1 from 1-hexadecyl-4-methylquinolinium bromide and the lithium salt of 2,3,5,6-tetrafluoro-TCNQ.

## EXAMPLE 4

The compound:

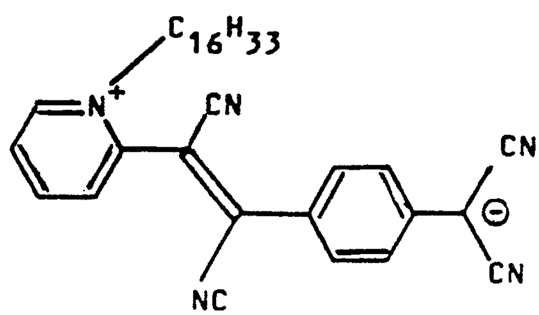

[λmax in acetonitrile = 645 nm compared with 595 nm for the corresponding compound with a -CH=C-(CN)- bridge;
elemental analysis: calculated C 78.6, H 7.9, N 13.5%; found C78.4, H 7.7, N 13.2%]
was prepared in a similar manner to that described in Example 1 from 1-hexadecyl-2-pyridiniumacetonitrile bromide and the lithium salt of TCNQ.

## Claims

1. A compound of formula (I);

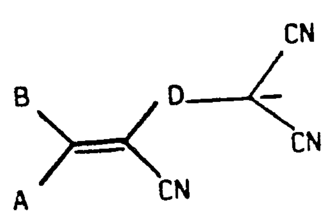

in which
A is

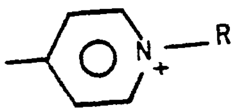 IIA

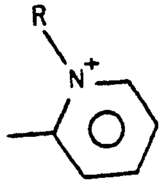 IIB

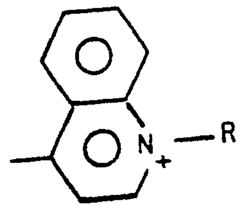 IIC

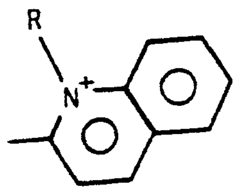 IID

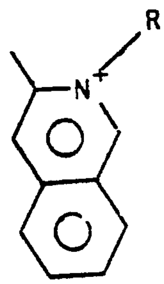 IIE

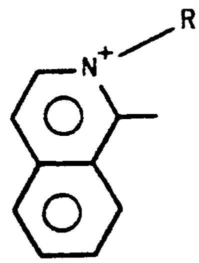 IIF

or

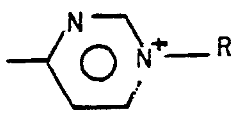 IIG

in which the pyridinium, quinolinium, isoquinolinium or pyrimidinium group is unsubstituted or substituted by at least one of F, Cl, Br, CN or $NO_2$ and R represents a straight hydrocarbon group containing at least 6 carbon atoms which is unsubstituted or substituted by fluorine and the chain of which may comprise a group -O-, -S-, -CO-O-, -O-CO-, -NH-CO-, -CO-NH- or carbon to carbon double or triple bond, or R represents a phenyl group substituted in the 3- or 4-position by a group R as hereinbefore defined or by a corresponding group containing less than 6 carbon atoms;
B is H, $CH_3$, F, Cl, Br, CN or $NO_2$; and D is

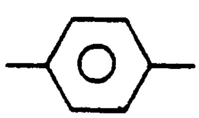

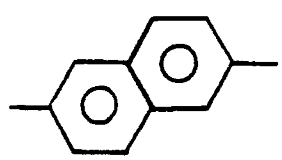

or

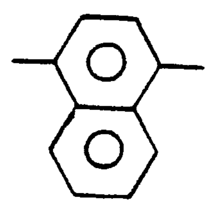

in which the phenyl group is unsubstituted or substituted by at least one of F, Cl, Br, CN or alkoxy of 1 to 4 carbon atoms.

2. A compound according to claim 1 in which A is other than a group of formula IIG, R represents a straight hydrocarbon group containing from 6 to 20 carbon atoms which is unsubstituted or substituted by fluorine, B is other than $CH_3$.

3. A compound according to claim 1 or 2 in which the pyridinium, quinolinium, isoquinolinium or pyrimidinium group is unsubstituted or substituted by at least one F atom.

4. A compound according to claim 1, 2 or 3 in which A is a pyridinium group and R is a straight hydrocarbon group containing 6 carbon atoms.

5. A compound according to claim 1, 2 or 3 in which A is a quinolinium group and R is a straight hydrocarbon group containing from 6 to 14 carbon atoms.

6. A compound according to any one of the preceding claims in which B is H or CN.

7. A compound according to any one of the preceding claims in which D is a phenyl group unsubstituted or substituted by at least one F atom or is a naphthyl group.

8. A compound according to claim 1 specifically identified herein.

9. A Langmuir-Blodgett film which comprises a compound of formula (I) as defined in any one of the preceding claims.

10. A film according to claim 9 which comprises two compounds of formula (I).

11. A film comprising two or more Langmuir-Blodgett films, each of which comprises a compound of formula (I) as defined in any one of claims 1 to 8 having a different switching frequency from the other compound(s) of formula (I).

12. An optical memory which comprises a film as defined in claim 9, 10 or 11.

13. A process for preparing a compound as defined in any one of claims 1 to 8 which comprises the reaction of a halide of the general formula:

**A - $CH_2$ - B. Hal**

(wherein Hal represents a halide and A and B are as defined in claim 1) and a compound of the formula:

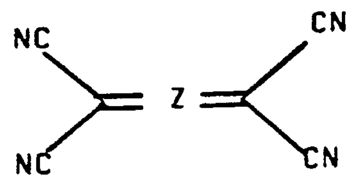

wherein Z represents:

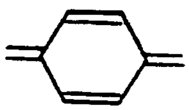 , 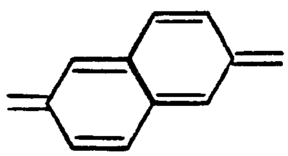 or 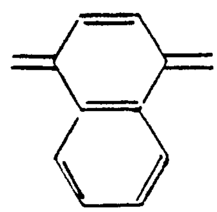

or an alkali metal salt thereof.

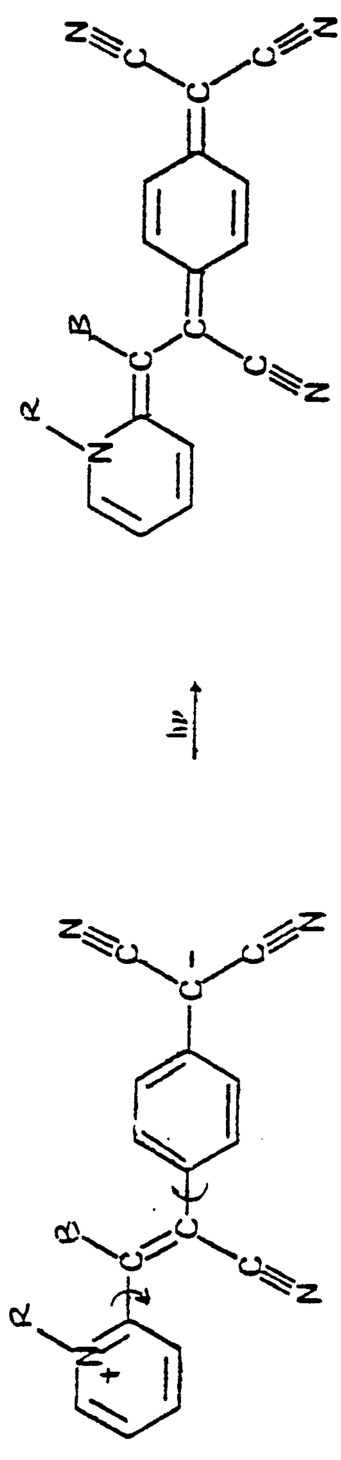


FIGURE 1

FIGURE 2

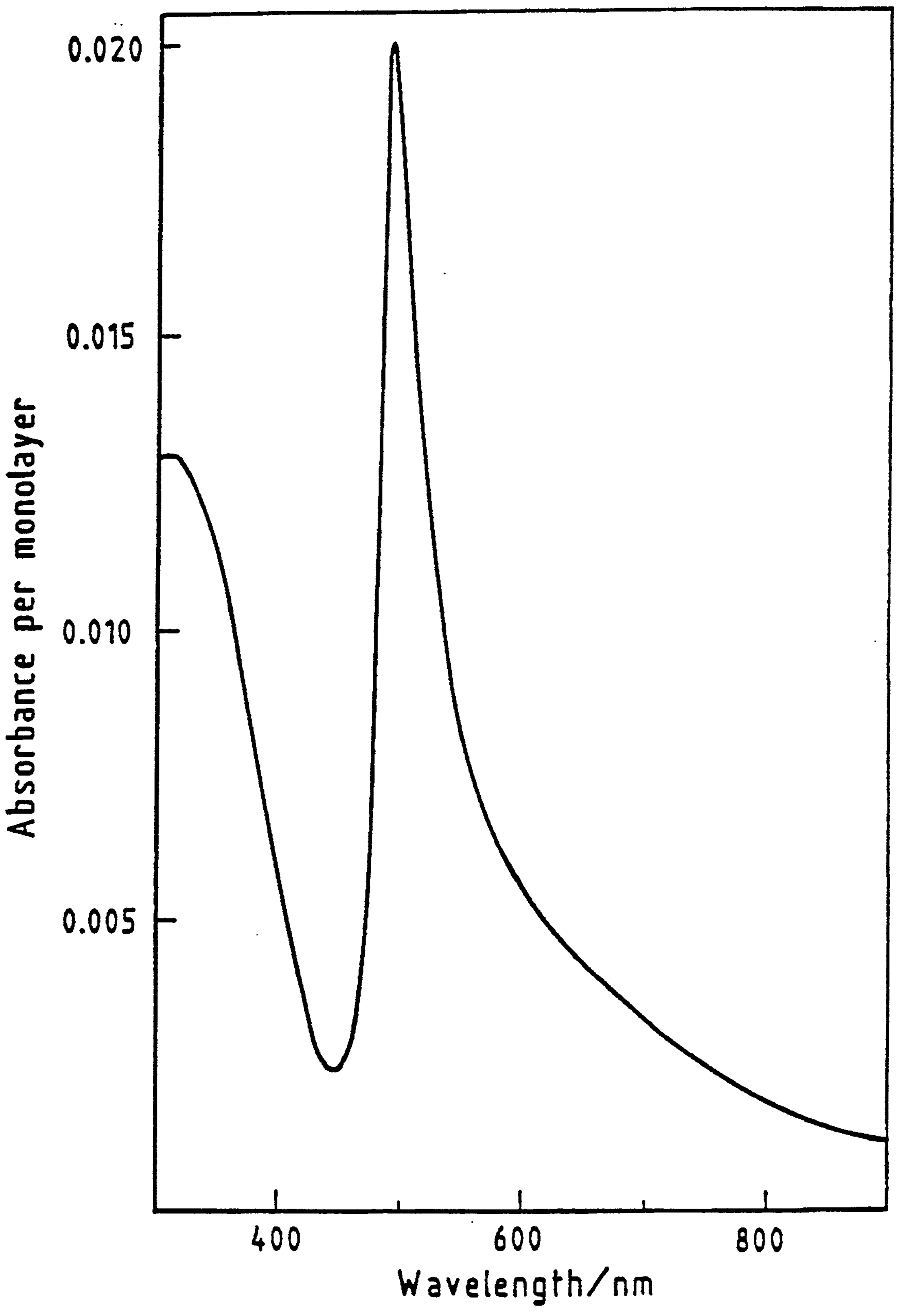
0.020
0.015
0.010
0.005
Absorbance per monolayer
400
600
800
Wavelength/nm

FIGURE 3

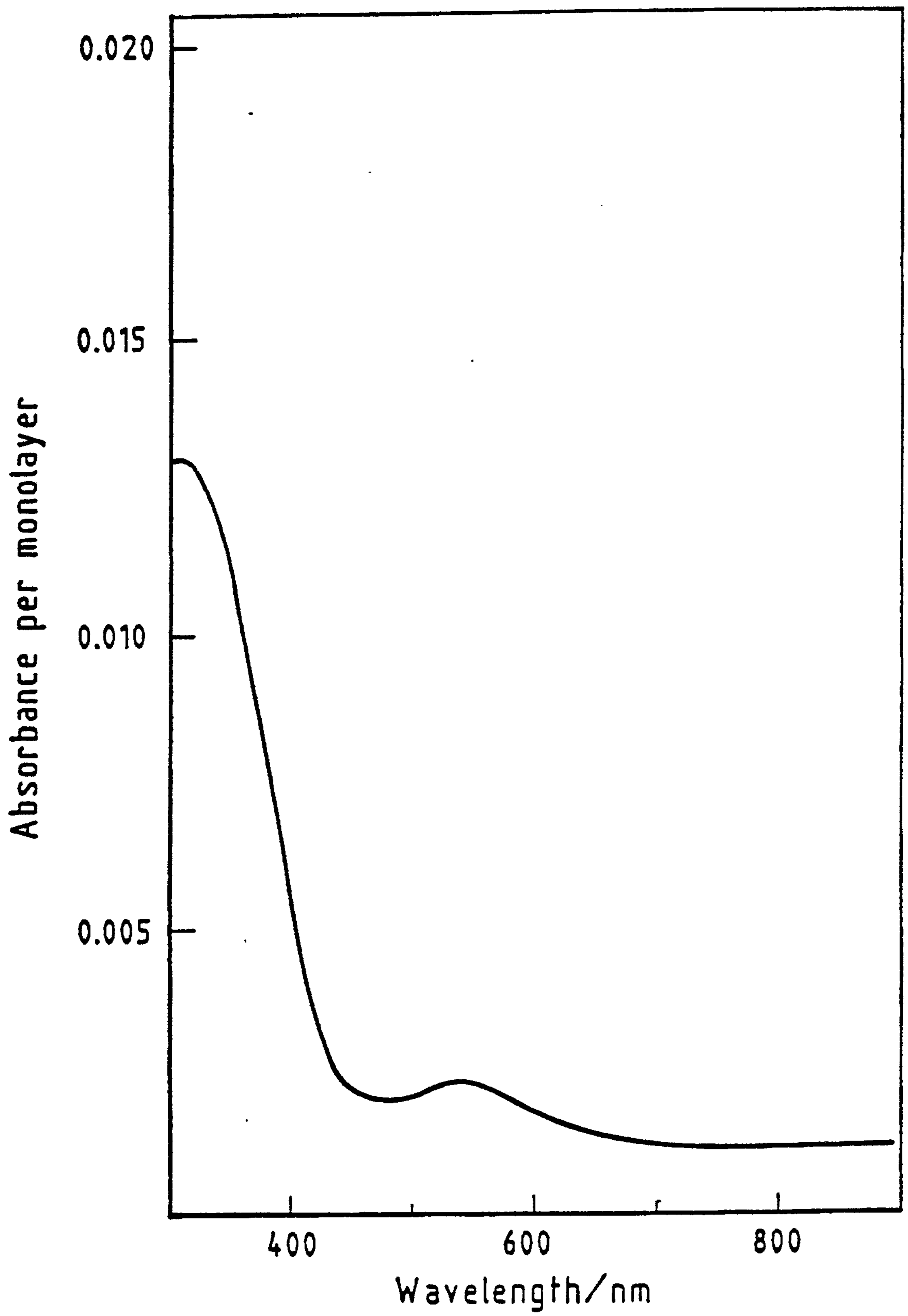
0.020
0.015
0.010
0.005
Absorbance per monolayer
400
600
800
Wavelength/nm

FIGURE 4

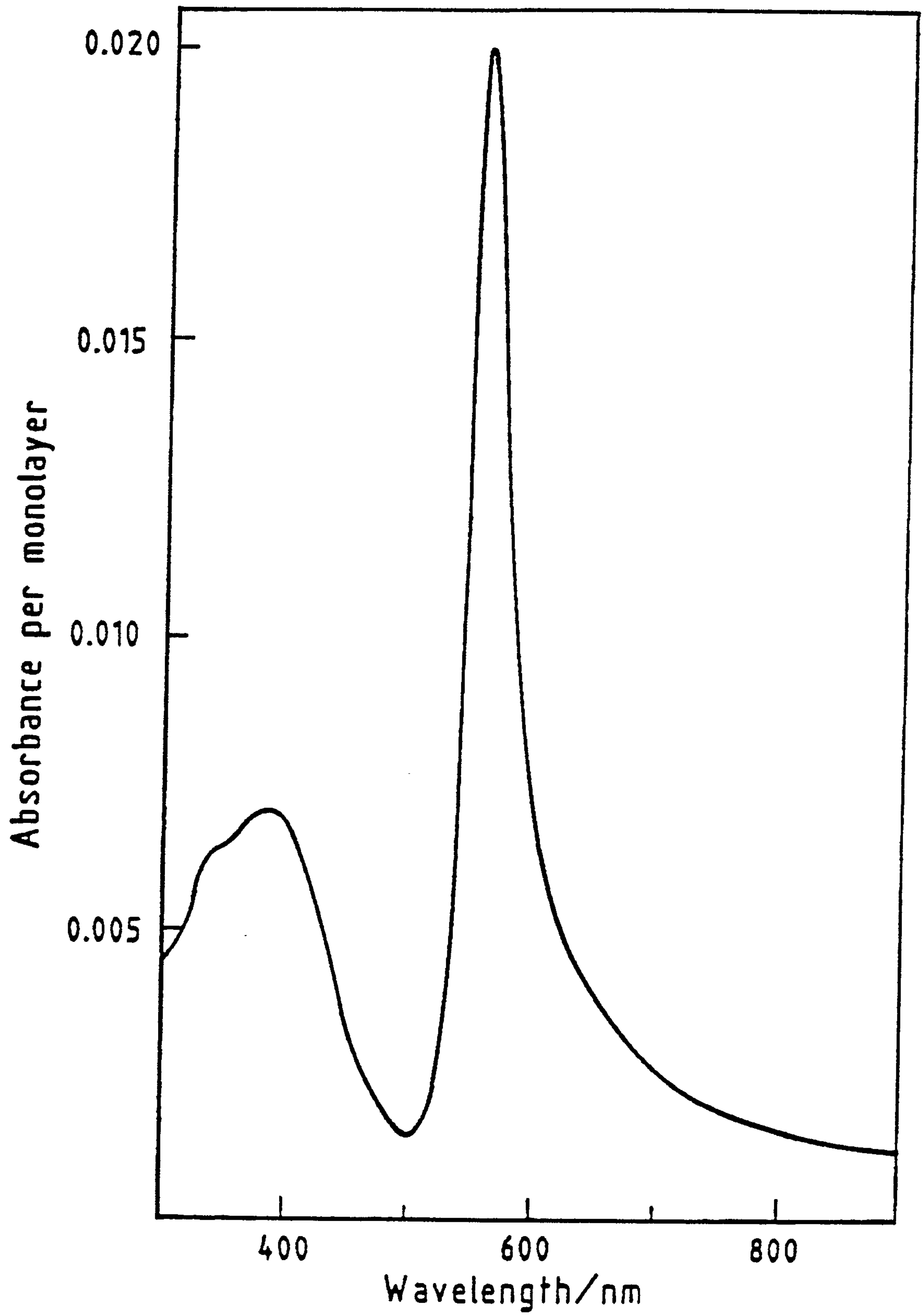
0.020
0.015
0.010
0.005
Absorbance per monolayer
400
600
800
Wavelength/nm

FIGURE 5

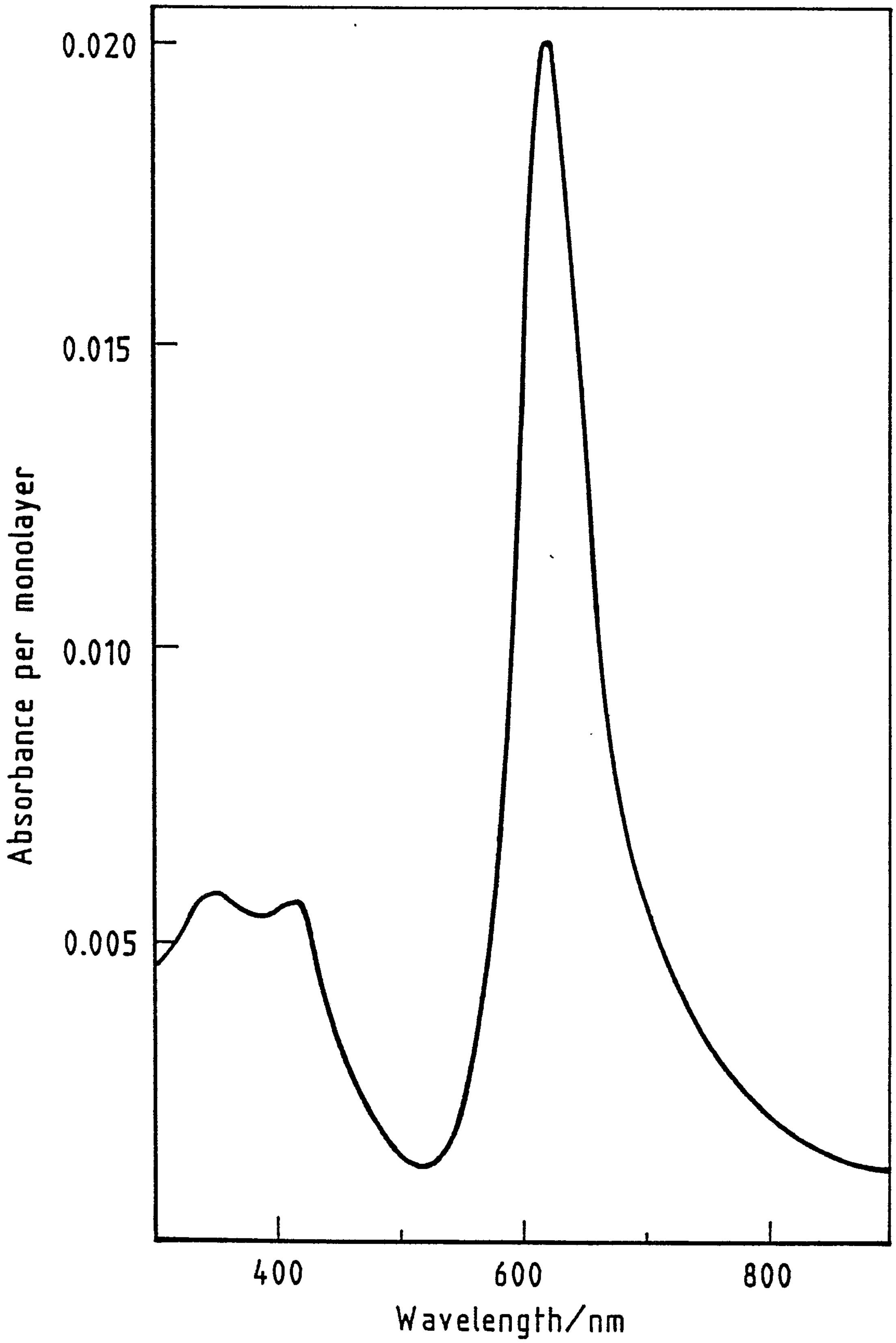
0.020
0.015
0.010
0.005
Absorbance per monolayer
400
600
800
Wavelength/nm

FIGURE 6

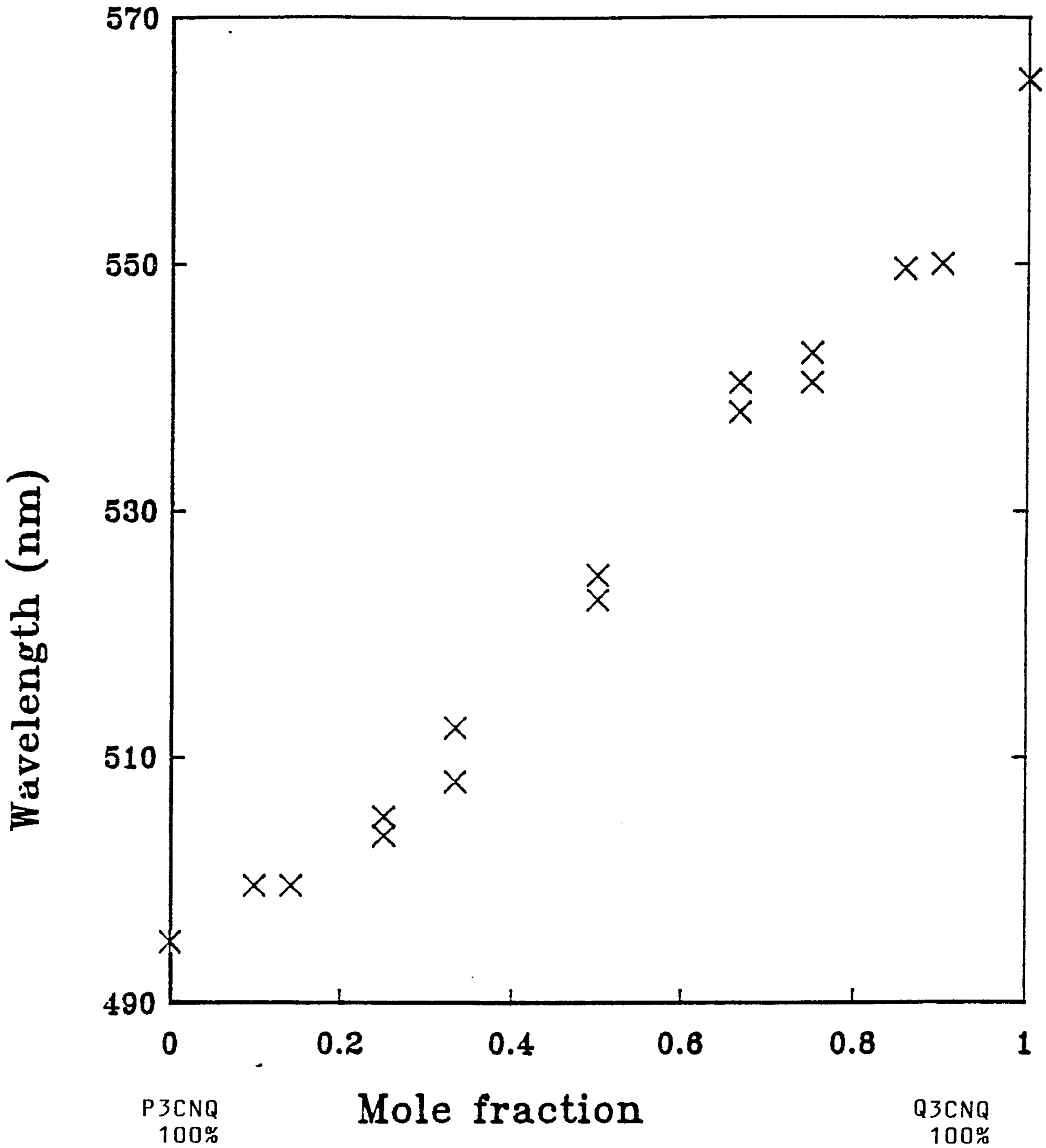
570
550
530
510
490
Wavelength (nm)
0
0.2
0.4
0.6
0.8
1
Mole fraction
P3CNQ
100%
Q3CNQ
100%

FIGURE 7

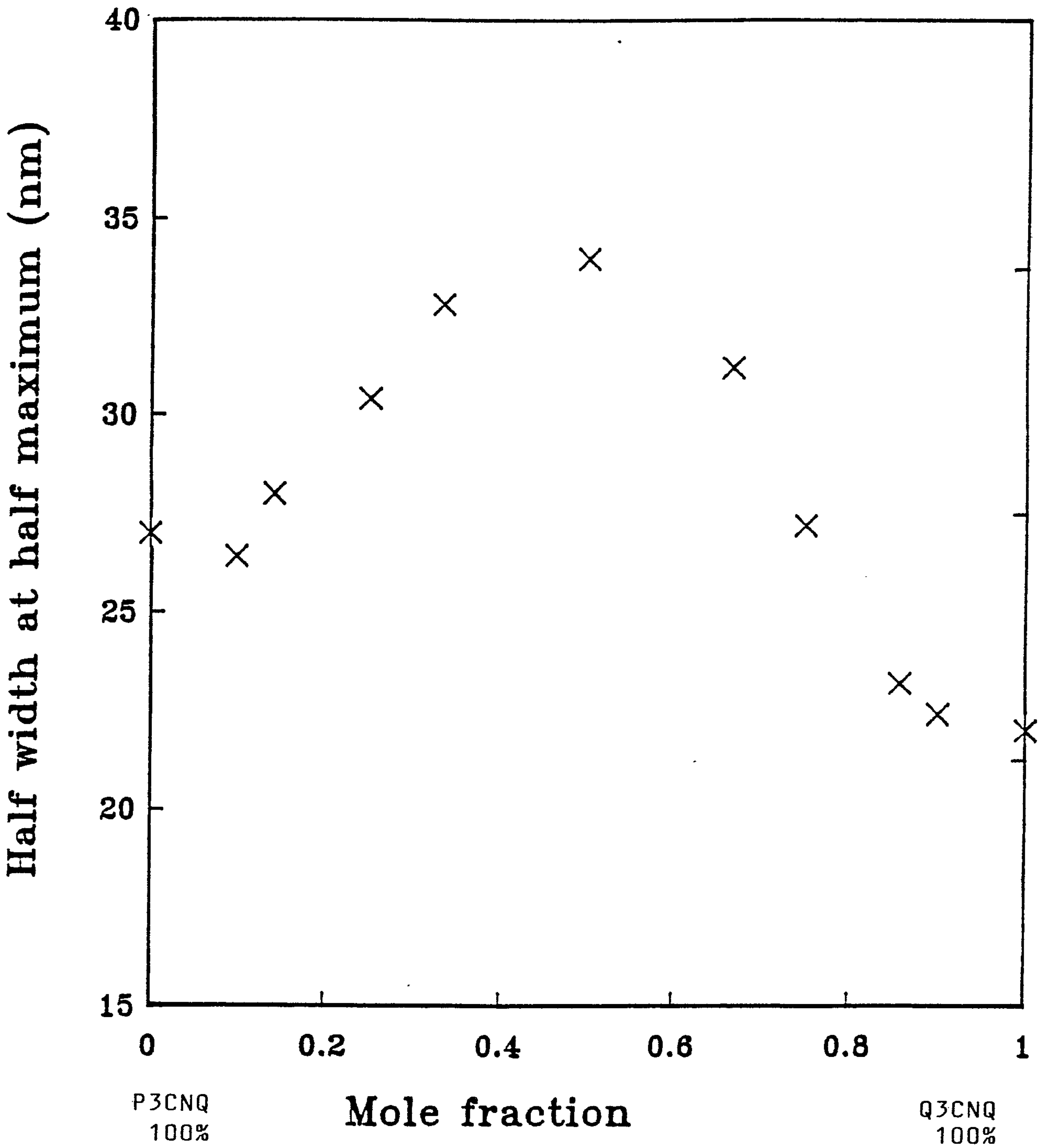
40
35
30
25
20
15
Half width at half maximum (nm)
0
0.2
0.4
0.6
0.8
1
P3CNQ
100%
Mole fraction
Q3CNQ
100%

European Patent Office

# EUROPEAN SEARCH REPORT

Application Number

EP 90 30 3473

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | MOLECULAR CRYSTALS AND LIQUID CRYSTALS. vol. 107, no. 1,2, 1984, MONTREUX CH pages 133 - 149; R.M.Metzger et al.: "Crystal and molecular structure and properties of picolyltricyanoquinododimethan,the zwitterionicdonor-pi-acceptor adduct between.." * page 135; figures 1, formula VII; page 140, figure 2; page 136, paragraph 3 - page 137, paragraph 1 * | 1-8, 13 | C 07 D 215/12<br>C 07 D 217/14<br>C 07 D 213/57<br>C 07 D 239/26<br>C 09 B 23/14<br>G 11 B 7/24 |
| A | EP-A-0 228 169 (CELANESE) * abstract; claims 1-20 * | 9-12 | |

TECHNICAL FIELDS SEARCHED (Int. Cl.5)

C 07 D
G 11 B 7
C 09 B

The present search report has been drawn up for all claims

| Place of search | Date of completion of the search | Examiner |
|---|---|---|
| THE HAGUE | 28-06-1990 | VANHECKE H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document